**Europäisches Patentamt**

**European Patent Office**

**Office européen des brevets**

(19)

(11) Veröffentlichungsnummer: **0 393 431**

**A1**

(12) # EUROPÄISCHE PATENTANMELDUNG

(21) Anmeldenummer: **90106529.2**

(22) Anmeldetag: **05.04.90**

(51) Int. Cl.5: **C07K 7/10, A61K 37/64, C12N 15/15**

(30) Priorität: **18.04.89 DE 3912638**

(43) Veröffentlichungstag der Anmeldung:
**24.10.90 Patentblatt 90/43**

(84) Benannte Vertragsstaaten:
**AT BE CH DE DK ES FR GB GR IT LI LU NL SE**

(71) Anmelder: **BAYER AG**

**D-5090 Leverkusen 1 Bayerwerk(DE)**

(72) Erfinder: **Bruns, Wolfgang, Dr.**
**Kaiser-Wilhelm Allee 37**
**D-5600 Wuppertal 1(DE)**
Erfinder: **Schnabel, Eugen, Dr.**
**Schimmelweg 6**
**D-5600 Wuppertal 11(DE)**
Erfinder: **von Wilcken-Bergmann, Brigitte, Dr.**
**Mauenheimerstrasse 70**
**D-5000 Koeln 80(DE)**

(54) **Gentechnologisch hergestellte Homologe des Alzheimer-Protease-Inhibitors, Wirtstämme sowie Expressionsvektoren für ihre Herstellung für ihre Verwendung als Arzneimittel.**

(57) Die vorliegende Erfindung betrifft Homologe des Alzheimer Protease Inhibitors (API), deren Aminosäuresequenzen sowie das Verfahren zu ihrer Herstellung mit gentechnologischen Methoden und ihre Verwendung als Arzneimittel.

EP 0 393 431 A1

## Gentechnologisch hergestellte Homologe des Alzheimer Protease Inhibitors, Wirtstämme sowie Expressionsvektoren für ihre Herstellung und ihre Verwendung als Arzneimittel

Die vorliegende Erfindung betrifft Homologe des Alzheimer Protease Inhibitors (API), deren Aminosäuresequenzen sowie das Verfahren zu ihrer Herstellung mit gentechnologischen Methoden und ihre Verwendung als Arzneimittel.

Amyloid-Protein-Aggregate wurden in neuritischen Plaques und cerebrovaskulären Ablagerungen bei Patienten mit Alzheimer'scher Krankheit gefunden (C.L. Masters et al., Proc. Nat. Acad. Sci USA 82, 4245-4249, 1985). Molekularbiologische Untersuchungen der entsprechenden cDNA aus humanem Gehirngewebe zeigten, daß das Amyloid-Protein Bestandteil eines Precursorproteins von 695 Aminosäuren ist (J. Kang et al., Nature 325, 733-736; 1987). Weiterführende Untersuchungen an cDNAs aus peripherem Gewebe führten zur Entdeckung von verwandten cDNA-Spezies, die sich durch ein zusätzliches DNA-Segment von 168 Basenpaaren (bp) (P. Ponte et al., Nature 331, 525-527 bzw. R.E. Tanzi et al., Nature 331, 528-530; 1988) bzw. 225 bp (N. Kitaguchi et al., Nature 331, 530-532; 1988) im Gen des Amyloid-Precursorproteins auszeichnen.

Die von dem 168 bzw. 225 bp DNA-Segment abgeleitete Aminosäuresequenz im Leseraster des Amyloid-Precursorproteins zeigt starke Homologie mit Proteaseinhibitoren vom Kunitztyp, wie z.B. den inhibitorisch aktiven Domänen des Inter-$\alpha$-Trypsin-Inhibitors (ITI) oder dem basischen pankreatischen Trypsin-Inhibitor (BPTI bzw. Aprotinin). Kitaguchi et al konnten Trypsin-inhibitorische Aktivität nach Transfektion von COS-1 Zellen mit der um 225 bp längeren cDNA des Amyloid-Precursors nachweisen. Die Funktion und Bedeutung des beschriebenen Alzheimer Protease Inhibitors (API) für die Entstehung des Amyloid-Plaques und für die Pathogenese der Alzheimer'schen Krankheit ist nicht bekannt.

Proteaseinhibitoren wie das dem API verwandte Aprotinin (Basischer Pankreatischer Trypsin Inhibitor aus Rinderorganen) besitzen eine breite inhibitorische Spezifität und werden seit vielen Jahren als Arzneimittel in der Therapie von Krankheiten verwendet, die sich unter anderem durch einen Mangel an natürlichen Proteaseinhibitoren auszeichnen (vgl. H. Fritz und G. Wunderer, Arzneimittelforschung 33, 479-494; 1983).

Zahlreiche Krankheiten sind nun aber bedingt durch eine Freisetzung von Enzymen, die nicht durch Inhibitoren wie Aprotinin gehemmt werden können. Dazu gehören lysosomale Enzyme wie z.B. die Granulozytenelastase. Normalerweise wird diese bei der Ausschüttung in den extrazellulären Raum inaktiviert durch Komplexierung mit körpereigenen Inhibitoren wie $\alpha_1$-Proteaseinhibitor ($\alpha_1$-PI) (J. Travis und G.S. Salvesen, Ann. Rev. Biochem. 655-709; 1983) durch Antileukoprotease (HUSI-I) (H.Schissler et al. in: Neutral Proteases of Human Polymorphonuclear Leukocytes (1978) 195-207; K. Havemann und A. Janoff Hrgb., Urban + Schwarzenberg, Baltimore) oder durch $\alpha_2$-Makroglobulin ($\alpha_2$-M) (G. Salvesen, D. Virca und J. Travis, Ann N.Y. Acad. Sci. 421, 316-326; 1983). Ein hereditiärer $\alpha_1$-PI-Mangel bzw. die oxidative Inaktivierung des Inhibitors (J. Travis und G.S. Salvesen, Ann. Rev. Biochem. 655-709; 1983) oder eine massive Enzymausschüttung, insbesondere von Granulozytenelastase, führen zu einem extensiven Abbau von Bindegewebe und humoralen Funktionsproteinen mit ernsthaften klinischen Symptomen wie Lungenemphysem, Schocklunge, Adult Respiratory Distress Syndrome, Gerinnungsstörungen sowie Nieren- und Leber-Versagen (vgl. M. Jochum et al. in: neue Wege in der Entzündungsdiagnostik, PMN-Elastase (1985), GIT-Verlag, Darmstadt; W.W. McGuire et al., J. Clin. Invest. 69, 543; 1982 sowie C.T. Lee et al., N. Engl. J. Med. 304, 192-196; 1981). Auch bei akuten und chronischen Entzündungen, z.B. bei der rheumatoiden Arthritis ist die verhängnisvolle Rolle der Granulozytenelastase gesichert (K. Klesiek et al. in: Neue Wege in der Entzündungsdiagnostik, PMN-Elastase (1985) 71-82, GIT-Verlag, Darmstadt).

In experimentellen Sepsis- und Emphysem-Modellen wurde die therapeutische Wirksamkeit von synthetischen Elastaseinhibitoren (J.C. Powers, Ann. Rev Respir. Dis. 127, 554-558; 1983) sowie dem gentechnologisch hergestellten Elastaseinhibitor Eglin c (H.P. Schnebli et al, Europ. J. Respir. Dis 66 Suppl. 66-70; 1985) nachgewiesen. Vor allem in der Langzeittherapie bietet jedoch der Einsatz eines stabilen und oxidationsunempfindlichen Elastaseinhibitors humaner Provenienz wegen des fehlenden Allergisierungsrisikos und der besseren Verträglichkeit erhebliche Vorteile.

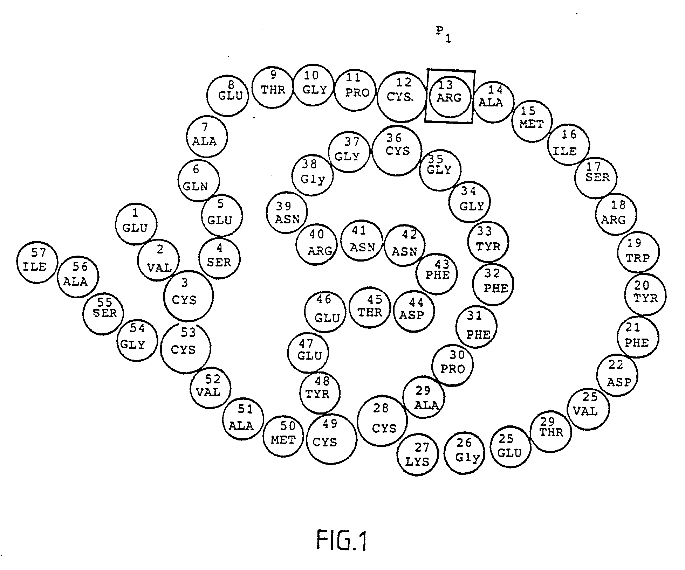

Es wurde gefunden, daß das Hemmspektrum von API durch Aminosäureaustausch in Position 13 (P1) (Abb. 1) mittels rekombinanter DNA-Technologie selektiv verändert werden kann und daß in Kombination mit Aminosäureaustauschen in Position 15 und 37 spezifische Inhibitoren gegen Serinproteasen wie z.B. Elastase, Kallikrein und Cathepsin G erhältlich sind.

Darüber hinaus sollte wegen des geringen Molekulargewichts von API-Homologen im Komplex mit der Leukozytenelastase eine effiziente Ausscheidung von Inhibitor und freigesetzter Elastase über die Nieren erfolgen.

Die Tatsache, daß das API-Protein humanen Ursprungs ist sowie sein niedriges Molekulargewicht lassen in der klinischen Anwendung von API-Homologen geringe Probleme hinsichtlich der Verträglichkeit und des Risikos allergischer Komplikationen erwarten.

Ein weiterer Vorteil von API-Nomologen nach Aminosäureaustausch von Met in Position 15 ist, im Vergleich zu $\alpha_1$-P1 und Antileukoprotease, deren Unempfindlichkeit gegenüber der oxidativen Inaktivierung bei entzündlichen Prozessen, bei denen stark oxidative Substanzen produziert und freigesetzt werden. Dadurch sollten, im Vergleich zu $\alpha_1$-PI und Antileukoprotease, geringere Dosen von API-Homologen notwendig sein, um einen vergleichbaren Schutz gegen Proteasen zu erreichen.

Ziel der Erfindung ist es, pharmakologisch nützliche Peptide herzustellen, die Proteaseinhibitionsaktivität mit veränderter Selektivität und verbesserter inhibitorischer Effizienz besitzen. Diese gentechnologisch hergestellten Peptide beinhalten die Aminosäuresequenz des Alzheimer Protease Inhibitors (API), wobei mindestens eine Aminosäure der natürlichen Sequenz durch eine andere natürliche Aminosäure ersetzt worden ist. Die erfindungsgemäßen Inhibitoren können außerdem veränderte N-terminale und/oder C-terminale Aminosäuresequenzen besitzen, ohne daß hierdurch ihre inhibitorische Spezifität verändert wäre.

Darunter sind zu verstehen: Deletionen einer oder mehrerer Aminosäuren oder Additionen von Aminosäuren, wie z.B. von Signalpeptiden oder von Met am $NH_2$-Terminus bzw. Addition von Oligopeptiden als Linkersequenzen am COOH- oder $NH_2$-Terminus, die konstruktionsbedingt sind oder mit deren Hilfe eine höhere Expression oder eine Verbesserung der Proteinreinigung erreicht werden kann. Desgleichen sind unter erfindungsgemäßen API-Homologen Inhibitoren mit Aminosäureaustauschen an Positionen von sauren (Asp, Glu) bzw. basischen (Arg, Lys) Aminosäureresten (ausgenommen $P_1$) zu verstehen, mit dem Ziel, bei unveränderter inhibitorischer Spezifität die Azidität und damit die pharmakokinetischen Eigenschaften des Inhibitors gezielt zu verändern. Gleiches kann auch durch Substitution neutraler Aminosäurereste in flexiblen Regionen des API durch basische oder saure Aminosäuren erzielt werden, beispielsweise Ersatz von Position 37-39 durch Arg-Ala-Lys, wodurch zusätzlich 2 basische Aminosäuren im API eingeführt werden können, ohne die Spezifität zu verändern.

Ganz allgemein werden unter erfindungsgemäßen Inhibitoren auch solche API-Varianten verstanden, die durch einen Aminosäureaustausch in Position 13 zu Inhibitoren der Leukozytenelastase, des Kallikreins oder des Cathepsin G wurden und zusätzlich einen, zwei oder auch mehrere weitere Aminosäureaustausche tragen, die diese Hemmspezifität nicht verändern.

In den erfindungsgemäßen, gentechnologisch hergestellten Homologen des API können neben einem veränderten Aminosäurerest im aktiven Zentrum $P_1$ (Position 13) zusätzlich ein oder mehrere Aminosäurereste in anderen Positionen im Molekül ausgetauscht sein. Solche bevorzugten Positionen für Austausche sind

Position 13 ($P_1$ = aktives Zentrum):
Val, Ile, Leu, Ala, Met, Asn, Gln, Phe, Tyr oder Trp
Position 15:
Leu, Gly, Ala, Phe, Arg, Val, Ile, Ser, Thr, Asp, Glu, Asn, Gln oder Tyr
Position 37:
Arg, Val, Ile, Leu, Met, Asn, Gln, Asp, Glu, Phe, Ser oder Thr
Position 50:
Val, Ile, Leu, Gln, Glu, Thr, Asp, Phe, Lys oder Arg.

Gegenstand der Erfindung sind außerdem die synthetischen Desoxyribonukieinsäuren (DNAs), die für die erfindungsgemäßen API-Homologen kodieren. Der Austausch einzelner Aminosäurereste ist bekanntermaßen durch ortsspezifische Mutagenase oder Synthese des entsprechenden Gens möglich. Methoden zur Expression der heterologen DNA in Mikroorganismen oder in eukaryotischen Zellen sind ebenso bekannt. So wurde gezeigt, daß Aprotinin oder Homologe in rekombinanten Mikroorganismen unter Verwendung synthetischer Gene entweder als Met-Aprotinin (B. v. Wilcken-Bergmann et al, EMBO J., 5, 3219; 1986) oder als Fusionsprotein mit $\beta$-Galaktosidase (E.A. Auerswald et al., Europ. Patentanmeldung 238 993 vom 26.3.1986) exprimiert werden können. Es ist weiterhin bekannt, daß Proteaseinhibitoren als Precursorproteine hergestellt werden können, wie z.B. Aprotinin mit der Signalsequenz der alkalischen Phosphatase (S. Anderson et al., Proc. Nat. Acad. Sci. USA 80, 6368; 1983) und der pankreatische, sekretorische Trypsin-Inhibitor (PSTI) mit der OMPA-Signalsequenz (F. Maywald et al., Gene 68, 357-369; 1988).

Eine andere Möglichkeit der Genexpression in Form von "inclusion bodies" ist für die Herstellung von humanem Aprotinin als Fusionsprotein mit einer Teilsequenz der MS2-DNA-Polymerase gezeigt worden (E. Schnabel et al., EP-A 297 362 bzw. DE-OS 3 724 570). Der gewünschte Proteaseinhibitor ist durch selektive, enzymatische oder chemische Spaltung des Fusionsproteins zugänglich, beispielsweise durch BrCN-Spaltung, wenn die Verknüpfung der Fusionspartner über ein Methionin erfolgt, und wenn gleichzeitig diese Aminosäure in dem Proteaseinhibitor abwesend ist bzw. ausgetauscht werden kann.

Es ist darüber hinaus auch möglich, die erfindungsgemäßen Proteaseinhibitoren in eukaryontischen Organismen zu exprimieren (z.B. Hefen, filamentöse Pilze etc.).

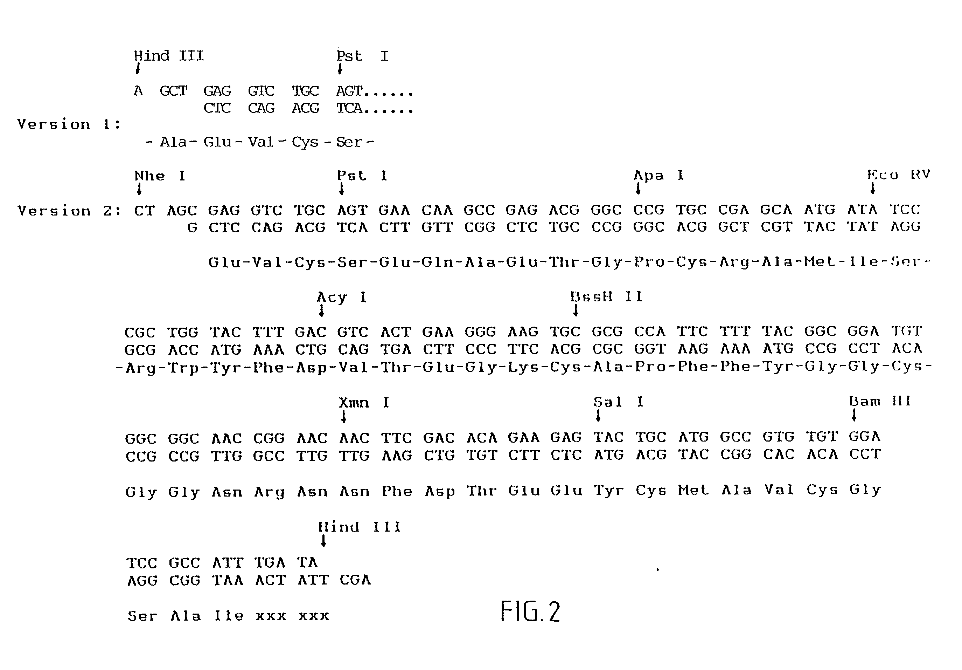

Die vorliegende Erfindung betrifft insbesondere die synthetische DNA-Sequenz in Abb. 2 und deren durch verschiedene Codons derselben Aminosäure abgeleiteten Äquivalente, bei deren Translation die erfindungsgemäßen Inhibitoren erhalten werden.

Abhängig von der Art des Expressionssystems können die erfindungsgemäßen Gene der API-Homologen zusätzlich ein Startcodon für Met oder eine DNA-Sequenz enthalten, die für ein Signalpeptid, ein Linkerpeptid oder ein Fusionsprotein kodiert.

Weiterhin betrifft die Erfindung einen Hefe-Expressionsvektor, in welchen die erfindungsgemäßen DNA-Sequenzen für die beanspruchten API-Homologen insertiert werden und der für die Transformation geeigneter Wirkstämme verwendet wird.

Je nach der Natur des Expressionssystems können die erfindungsgemäßen Gene für die Expression der beanspruchten Proteinaseinhibitoren zusätzlich ein Start-Codon für Met oder eine Nukleotidsequenz vorgeschaltet haben, die für ein Signalpeptid kodiert.

Bevorzugte Fusionspartner für die beanspruchten Proteaseinhibitoren können sekretorische Signalsequenzen von mikrobiellen Proteinen sein, wie z.B. omp A (Ghrayed u.a. EMBO Journal 3, 2437-2442; 1984) oder pho A (Gray u.a., Gene 39, 247-254; 1985). Im Falle der Expression in Hefen kann es die Signalsequenz des mating factorsalpha (MFα) sein (Kurjan, Cell 30, 933; 1982).

Die Fusionsproteine werden während der Fermentation in das Periplasma oder Kulturmedium sezerniert und nach an sich bekannten Verfahren aus den Kulturfiltraten isoliert. Bei der Wahl von geeigneten sekretorischen Systemen werden die Proteaseinhibitoren als native Proteine sezerniert. Eine Renaturierung nach der Bromcyan-Spaltung (E. Gross und B. Witkop, J. Amer. Chem. Soc. 83, 1510-1511; 1961) entfällt für den Fall einer korrekten Prozessierung beim Membrandurchtritt. Die erfindungsgemäßen Proteinaseinhibitoren werden aus den Reaktionsgemischen nach an sich bekannten Verfahren gereinigt und charakterisiert.

Eine alternative Möglichkeit der Gen-Expression ist im Falle der erfindungsgemäßen Proteinaseinhibitoren realisierbar, wenn die Fusionsproteine in der Zelle unlöslich in Form von "inclusion bodies" ausgeschieden werden. Solche Einschlüsse von unlöslichen Fusionsproteinen können erzeugt werden z.B. durch Fusionierung der API-Gene mit einer DNA-Teilsequenz der DNA-Polymerase des Bacteriophagen MS2 - E. Remault, P. Stanssens und W. Fiers, Gene 15, 81 (1981) und durch Fusion der Inhibitorgene mit einer Teilsequenz vom cII-Gen des Bacteriophagen Lambda (K. Nagai und H.G. Thorgersen, Nature 309, 810; 1984).

Die Produktion der erfindungsgemäßen Proteaseinhibitoren über Einschlußkörper umfaßt die folgenden Schritte:

1. Anzucht und Kultivierung des Wirtsstammes unter geeigneten Bedingungen;
2. Isolierung der Einschlußkörper aus den Wirtszellen;
3. Aufreinigung der Fusionsproteine;
4. Spaltung der Fusionsproteine;
5. Renaturierung der Inhibitoren;
6. Reinigung und Charakterisierung der Inhibitoren.

Für die Reinigung der löslichen Fusionsproteine kann es vorteilhaft sein, in das abzuspaltende Fusionspeptid gehäuft saure oder basische Aminosäuren einzubauen, so daß die Abtrennung durch Ionenaustauschchromatographie erleichtert wird.

Die Endreinigung der erfindungsgemäßen Proteaseinhibitoren erfolgt nach an sich bekannten Verfahren, wie z.B. Gelfiltration, Ionenaustauschchromatographie oder Affinitätschromatographie und Elektrophorese.

Derzeitig kennt man eine große Zahl von verschiedenen Mikroorganismen, die für die Transformation geeignet sind und die in Kulturen oder Fermentationsbrühen gezüchtet werden können. Bevorzugte Organismen zur Transformation schließen Bakterien, Hefen und Pilze ein.

Die vorliegende Erfindung umfaßt pharmazeutische Zubereitungen, die außer nicht-toxischen, inerten, pharmazeutisch geeigneten Exzipienten eine oder mehrere Verbindungen gemäß der Erfindung umfassen oder aus einer oder mehreren aktiven Verbindungen gemäß der Erfindung bestehen, sowie Verfahren zur Herstellung dieser Zubereitungen.

Die vorliegende Erfindung umfaßt auch pharmazeutische Zubereitungen in Dosiseinheiten. Dies bedeutet, daß die Zubereitungen in Form individueller Teile vorliegen, z.B. als Tabletten, beschichtete Tabletten, Kapseln, Pillen, Suppositorien und Ampullen, deren Gehalt an Wirkstoff einer Fraktion oder einem Vielfachen einer individuellen Dosis entspricht. Die Dosiseinheiten können z.B. enthalten: ein, zwei, drei oder vier individuelle Dosen oder eine Hälfte, ein Drittel oder ein Viertel einer individuellen Dosis. Eine individuelle Dosis enthält vorzugsweise die Menge an Wirkstoff, die bei einer Verabreichung gegeben wird, und die gewöhnlich dem Ganzen, der Hälfte oder einem Drittel oder einem Viertel der Tagesdosis entspricht.

Unter nicht-toxischen, inerten, pharmazeutisch geeigneten Exzipienten sind zu verstehen: feste, halbfeste oder flüssige Verdünner, Füller und Formulierungshilfsmittel aller Art.

Tabletten, beschichtete Tabletten, Kapseln, Pillen, Granalien, Suppositorien, Lösungen, Suspensionen und Emulsionen, Pasten, Salben, Gele, Cremes, Lotionen, Pulver und Sprays können als bevorzugte pharmazeutische Zubereitungen genannt werden.

Tabletten, beschichtete Tabletten, Kapseln, Pillen und Granalien können den Wirkstoff oder die Wirkstoffe zusammen mit üblichen Exzipienten, wie (a) Füller und Streckmittel, z.B. Stärken, Lactose, Sucrose, Glucose, Mannit und Siliziumoxid, (b) Bindemittel, z.B. Carboxymethylcellulose, Alginate, Gelatine und Polyvinylpyrrolidon, (c) anfeuchtende Mittel, z.B. Glycerin, (d) disintegrierende Mittel, z.B. Agar-Agar, Kalziumcarbonat und Natriumcarbonat, (e) Lösungsverzögerer, z.B. Paraffin und (f) Absorptionsbeschleuniger, z.B. quaternäre Ammoniumverbindungen, (g) Benetzungsmittel, z.B. Cetylalkohol und Glycerinmonostearat, (h) Absorbentien, z.B. Kaolin und Bentonit und (i) Schmiermittel, z.B. Talk, Kalzium- und Magnesiumstearat und feste Polyethylenglykole, oder Gemische der unter (a) bis (i) aufgeführten Substanzen, enthalten.

Die Tabletten, beschichteten Tabletten, Kapseln, Pillen und Granalien können mit üblichen Überzügen und Umhüllungen versehen werden, die gegebenenfalls Opazifizierungsmittel enthalten; sie können eine Zusammensetzung aufweisen, so daß die Freisetzung des Wirkstoffes ·oder der Wirkstoffe nur, oder vorzugsweise, in einem bestimmten Teil des Intestinaltraktes, gegebenenfalls in verzögerter Form, erfolgt, wobei Beispiele für geeignete Zubereitungen zum Einbetten polymere Substanzen und Wachse bilden.

Die aktive Verbindung oder Verbindungen, gegebenenfalls zusammen mit einem oder mehreren der vorstehend genannten Exzipienten, kann bzw. können auch in mikroverkapselter Form vorliegen.

Suppositorien können außer dem Wirkstoff bzw. den Wirkstoffen enthalten: übliche wasserlösliche oder wasserunlösliche Exzipienten, z.B. Polyethylenglykole, Fette, z.B. Kakaofett und höhere Ester (z.B. $C_{14}$-Alkohol mit $C_{16}$-Fettsäure) oder Gemische dieser Substanzen.

Salben, Pasten, Cremes und Gele können zusätzlich zu dem Wirkstoff oder den Wirkstoffen übliche Exzipienten enthalten, z.B. tierische und pflanzliche Fette, Wachse, Paraffine, Stärke, Tragacanth, Cellulosederivate, Polyethylenglykole, Silikone, Bentonite, Siliziumdioxid, Talk und Zinkoxid, oder Gemische dieser Substanzen.

Pulver und Sprays können außer dem Wirkstoff oder den Wirkstoffen übliche Exzipienten enthalten, z.B.: Lactose, Talk, Siliziumdioxid, Aluminiumhydroxid, Kalziumsilikat und Polyamidpulver, oder Gemische dieser Substanzen. Sprays können außerdem die üblichen Treibmittel enthalten, z.B. Chlorfluorkohlenwasserstoffe.

Lösungen und Emulsionen können außer dem Wirkstoff oder den Wirkstoffen übliche Exzipienten umfassen, wie Lösungsmittel, solubilisierende Agentien und Emulgiermittel, z.B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsamenöl, Erdnußöl, Maiskeimöl, Olivenöl, Rizinusöl und Sesamöl, Glycerin, Glycerinformal, Tetrahydrofurfurylalkohol, Polyethylenglykole und Fettsäureester von Sorbitan, oder Gemische dieser Substanzen.

Zur parenteralen Verabreichung können die Lösungen und Emulsionen auch in einer sterilen Form vorliegen, die mit Blut isoton ist.

Suspensionen können zusätzlich zum Wirkstoff oder den Wirkstoffen übliche Exzipienten enthalten, wie flüssige Verdünner, z.B. Wasser, Ethylalkohol oder Propylenglykol, Suspendierungsmittel, z.B. ethoxylierte Isostearylalkohole, Polyoxyethylensorbit und Sorbitanester, mikrokristalline Cellulose, Aluminium-metahydroxid, Bentonit, Agar-Agar und Tragacanth, oder Gemische dieser Substanzen.

Die genannten Formulierungsformen können auch enthalten: Farbstoffe, Konservierungsmittel und Additive, die den Geruch oder Geschmack verbessern, z.B. Pfefferminzöl und Eukalyptusöl, und Süßungsmittel, z.B. Saccharin.

Die therapeutisch wirksamen Verbindungen sollten in den vorstehend genannten pharmazeutischen Zubereitungen vorzugsweise in einer Konzentration von 0,1 bis 99,5 Gew.-%, vorzugsweise von ca. 0,5 bis 95 Gew.-%, bezogen auf das Gesamtgemisch, vorliegen.

Die vorstehend genannten pharmazeutischen Zubereitungen können zusätzlich zu den Verbindungen gemäß der Erfindung auch andere pharmazeutische Wirkstoffe enthalten.

Die vorstehend genannten pharmazeutischen Zubereitungen werden in üblicher Weise nach bekannten Verfahren hergestellt, z.B. durch Vermischen des Wirkstoffes oder der Wirkstoffe mit dem Exzipienten oder den Exzipienten.

Die Wirkstoffe oder die pharmazeutischen Zubereitungen können lokal, oral, parenteral, intraperitoneal und/oder rektal verabreicht werden, vorzugsweise erfolgt dies oral oder parenteral, wie z.B. intravenös oder

EP 0 393 431 A1

intramuskulär.

Im allgemeinen hat es sich sowohl in der Humanmedizin als auch in der Tiermedizin als vorteilhaft erwiesen, den Wirkstoff oder die Wirkstoffe gemäß der Erfindung in den Gesamtmengen von ca. 0,5 bis ca. 500, vorzugsweise von 4 bis 100 mg/kg Körpergewicht alle 24 Stunden, gegebenenfalls in Form von mehreren individuellen Verabreichungen, zu geben, um die besten Ergebnisse zu erzielen. Eine individuelle Verabreichung enthält den Wirkstoff oder die Wirkstoffe gemäß der Erfindung vorzugsweise in Mengen von ca. 1 bis ca. 250, insbesondere 3 bis 60 mg/kg Körpergewicht. Es kann jedoch erforderlich sein, von der genannten Dosis abzuweichen, insbesondere in Abhängigkeit von der Natur und dem Körpergewicht des zu behandelnden Individuums, der Natur und Schwere der Krankheit, der Art der Zubereitung und der Verabreichung der Medizin, und der Zeit oder dem Intervall, über den die Verabreichung stattfindet.

So kann es in einigen Fällen genügen, mit weniger als der vorstehend genannten Menge an Wirkstoff auszukommen, während in anderen Fällen die vorstehend genannte Menge an Wirkstoff überschritten werden muß. Die speziell erforderliche optimale Dosis und die Verabreichungsart der Wirkstoffe kann in einfacher Weise vom Fachmann auf der Basis seines Fachwissens entschieden werden.

Die erfindungsgemäßen Wirkstoffe sind sehr gut zur Behandlung von akuten Entzündungen, wie rheumatoider Arthritis, hereditäres angioneurotisches Ödem, Pneumonie, Pankreatitis und von Schockzuständen einsetzbar. Weiterhin haben die Lys-13- sowie die Arg-13-Varianten eine protektive Funktion bei Dialysen mit Hilfe von künstlichen Membranen und allgemein bei der extrakorporalen Zirkulation (open heart surgery). Klinische Befunde zeigen, daß durch Inhibitoren von Plasma-Kallikrein und Plasmin die durch Fibrinolyse und Koagulation bedingten Blutverluste bei "open heart surgery" vermindert werden können, S. 1289-1291; 1987 und C.F Scott et al, Blood, Vol. 69, 1431-1436; 1987).

## Material und Methoden

## A. Konstruktion der synthetischen Gene und der rekombinanten Klonierungs- und Expressionsvektoren

## Materialien:

## Reagentien und Enzyme

Reagentien für molekularbiologische und mikrobiologische Experimente wurden von Biolabs, BRL, Pharmacia, Boehringer (Mannheim), Difco, Merck, Serva Sigma und Biorad bezogen.

## Radioisotope

Adenosin-5'-$\alpha$[$^{35}$S]-thiotriphosphat wurde von Amersham Buchler bezogen.

## DNA

Plasmid- und Phagen-DNA sowie DNA-Linker für die Gen- und Vektorenkonstruktionen wurden bis auf nachfolgende Ausnahmen von Boehringer, Biolabs, BRL und Pharmacia bezogen.

## Ausnahmen:

Plasmid pUR 278: U. Rüther und B. Müller-Hill, EMBO Journal 2, 1791-1794 (1983); Plasmid pEx 31.b.: E. Beck, EMBL Heidelberg.

## E.coli-Stämme

E.coli RR1ΔM15 (ATCC Nr. 35102): U. Rüther, Nucleic Acids Res. 10, 5765-5772 (1982).

6

E.coli 537-W6, Lambda rex: W. Fiers, Universität Gent, Belgien (E. Remault u.a. (1981), Gene 15, 81-93).

## Medien und Antibiotika

Die Rezepturen für LB-, M9s- und Kappa 1776-Medium entsprechen den Angaben in: T. Maniatis u.a. - Molecular Cloning, Gold Spring Harbor, USA (1982) -. Für Agar-Platten wurde den Medien 15 g Bacto-Agar hinzugefügt. Für die Herstellung von Selektionsmedien wurden folgende Antibiotika verwendet: Chloramphenicol und Kanamycin von Boehringer; Ampicillin und Tetracyclin von Serva.

## Methoden

Die Routine-Techniken für molekulare Klonierungsexperimente, wie Isolierung und Aufreinigung von Plasmid-DNA, Restriktionsanalysen, Auftrennung und Isolierung von DNA-Fragmenten auf Agarose- und Acrylamidgelen, Transformation von Bakterien etc. sind beschrieben in: T Maniatis u.a., Molecular Cloning, Gold Spring Harbor (1982 bzw. 1988).

## Chemische Synthese von DNA-Oligonukleotiden

Die für Konstruktion der Proteaseinhibitorgene benötigten DNA-Oligonukleotide wurden mit einer automatischen DNA-Synthese-Maschine von Applied Biosystems, Model 380, synthetisiert. Die Aufreinigung der einzelsträngigen DNA-Oligonukleotide erfolgte über HPLC-Säulen oder durch präparative Acrylamidgelelektrophorese.

## DNA-Sequenzierung

DNA-Fragmente wurden in pUC-Vektoren kloniert und die DNA-Sequenz nach einem Protokoll von Boehringer Mannheim (Leitfaden zum schnellen und einfachen Plasmid-Sequenzieren; 1986) analysiert.

## Standard Expression der API-Homologen

Zur Expression der API-Homologen wurden sowohl Sekretionsvektoren bzw. Expressionsvektoren zur Produktion von cytoplasmatischen Einschlußkörpern verwendet, wie beschrieben von F. Maywald et al. Gene 68, 357-369 (1988) bzw. E. Schnabel et al., EP-A 297 362 und DE-OS 3 724 570.

Die rekombinanten Sekretionsvektoren wurden in den E.coli Stamm RR1ΔM15 eingeschleust, der MS2-Polymerase-Vektor in den E.coli Stamm 537.

## a) Induktion der Sekretionsvektoren

Frische Übernachtkulturen der transformierten E.coli Stämme wurden 1:100 im Medium (3 % beef extract, 1,5 % yeast extract, 0,5 % $K_2HPO_4$) verdünnt.

Die Kulturen wurden bei 28 °C oder 37 °C und 200 UpM bis zu einer $OD_{550nm}$ = 1,0 in Erlenmeyerkolben geschüttelt. Die Induktion des lac-Promoters erfolgte durch Zugabe von 1 mM IPTG (Isopropylthiogalactosid).

Nach 16 bis 24 Stunden Induktion bei 37 °C wurden die Kulturen mittels Zentrifugation geerntet (10 Minuten; 15 bis 20.000 g; 4 °C). Bei Verwendung der Sekretionsvektoren wurden die Kulturüberstände anhand der enzyminhibitorischen Aktivität auf den Gehalt der sezernierten API-Homologen getestet (s. Methodenteil B).

## b) Induktion des $\lambda P_L$-MS2-Polymerase Vektors

Die Expression der API-Homologen als Fusionsproteine mit einer Teilsequenz der DNA-Polymerase des Phagen MS2 erfolgte mit einem pEx-Vektor (E. Beck, EMBL-Heidelberg) unter Kontrolle des $P_L$-Promoters des Phagen Lambda.

Die Anzucht der transformierten E.coli Stämme (Derivate von Ec 537) erfolgte in 10 ml bzw. 1 L LB-Medium mit 100 µg/ml Ampicillin bei 200 UpM und 28° C. Bei einer Wuchsdichte von $OD_{550nm} = 1,0$ wurde die Temperatur auf 42° C erhöht (Hitzeinduktion) und die Kulturen für weitere 3 Stunden bei 200 UpM inkubiert.

Die Zellen wurden durch Zentrifugation geerntet (s.o.), in SDS-Probenpuffer aufgeschlossen und Aliquots auf SDS-Polyacrylamidgelen durch Elektrophorese aufgetrennt.

Die in Form von Einschlußkörpern akkumulierten Fusionsproteine wurden durch Färbung der Gele mit Coomassie Blau nachgewiesen und der prozentuale Anteil der Fusionsproteine densitometrisch gemessen.

B) Isolierung und Charakterisierung der erfindungsgemäßen Proteaseinhibitoren

Materialien

Enzyme

Humane Granulozytenelastase (SE 563) und humanes Cathepsin G (SG 45) wurden von Elastin Products Company Inc., P.O. Box 147, Pacific, Ma. 63069, USA bezogen; Trypsin (Rind) von E. Merck, Darmstadt.

Humanes Plasmakallikrein (80-13-1101) stammt von der Fa. Protogen AG, Weidenmattweg 4, Postfach, CH-4448 Läufelfingen, Schweiz. Humanes Harnkallikrein wurde uns von Dr. R. Geiger (Abtig. f. Klinische Chemie und Klinische Biochemie in der Chirurgischen Klinik, Universität München, Nußbaumstr 20, 8000 München 2) überlassen; human Plasmin wurde von KABI, AB, Stockholm gekauft; humanes anionisches und kationisches Trypsin von Dr. K. Ohlsson (Dept. Clin. Chemistry and Surgery, Malmö General Hospital, S-21401 Malmö, Schweden).

Substrate

Suc-Ala-Ala-Val-PNA und Meosuc-Ala-Ala-Pro-Val-pNA (HGE) sowie Meosuc-Ala-Ala-Pro-Met-pNA (HCG) wurden von der Fa. Bachem, Feinchemikalien AG, Hauptstr. 144, CH-4416 Bubendorf, Schweiz bezogen; D-Pro-Phe-Arg-pNAx2HCl = S-2302 (Plasmakallikrein), D-Val-Leu-Arg-pNAx2HCl = S-2260 (Qrgankallikrein) und Pyr-Gly-Arg-pNAxHCl = S-2444 (Trypsin) wurden von der Deutschen Kabi GmbH, Levelingstr. 18, D-8000 München 80 gekauft. Tos-Gly-Pro-Lys-pNA•CH₃-COOH (Chromozym PL) (Plasmin) stammte von Boehringer, Mannheim.

HPLC und FPLC

Präparative und analytische HPLC- sowie FPLC-Chromatografien wurden mit geeigneten Säulen durchgeführt.

Aminosäuresequenzbestimmungen

Für die Bestimmung der Aminosäuresequenzen der erfindungsgemäßen Proteinaseinhibitoren wurden 0,5 bis 2 nMol Substanz auf ein mit Polybren® vorbehandeltes Glasfaserfilter geladen und die Sequenzanalyse mit dem Gasphasen Protein Sequenzer (Fa. Applied Biosystems 470) durchgeführt. Die in jedem Cyclus freigesetzten Aminosäure-phenylthiohydantoine wurden mittels HPLC an einer Zorbax CN-Säule durch isokratische Elution nach Beyreuther (K. Beyreuther, B. Biesler, J. Bovens, R. Dildrop, K. Neifer, K. Stüber, S. Zais, R. Ehring und P. Zabel in: Modern Methods in Protein Chemistry S. 303-325; 1983, Walter de Gruyter, Berlin) identifiziert und quantifiziert.

## Bestimmung der quantitativen Aminosäurezusammensetzung

Jeweils ca. 1 nMol Inhibitor wurde nach J.T. Potts jr. - Anal. Biochem. 131, 1-15 (1969) - mit 200 $\mu$l konstantsiedender Salzsäure, die 0,05 % Mercaptoethanol enthielt, in einem abgeschmolzenen evakuierten Pyrexröhrchen 22 Stunden auf 110° C erhitzt. Die Eindampfrückstände wurden in 150 $\mu$l 0,2 M Natriumcitratpuffer pH 2,2 aufgenommen und unlösliche Anteile durch Filtration abgetrennt. Die in den Hydrolysaten vorliegenden Aminosäuren wurden in einem Biotronic LC 5000 Aminosäureanalysator getrennt, der mit einem Fluoreszensdetektor und einem Shimadzu CR 2AC-Integrator ausgerüstet war. Die Quantifizierung erfolgte nach Derivatisierung mit o-Phthaldialdehyd, wie von J.R. Benson und P.E. Hare - Proc. Nat. Acad. Sci. 72, 619-622 (1975) -beschrieben.

## Polyacrylamidaelelektrophoresen

Die SDS-Gelelektrophoresen wurden nach Laemmli -U.K. Laemmli, Nature 277, 680 (1970) - durchgeführt. Die Proteinbanden wurden angefärbt, wie von J.L. Stephano, M. Gould und L. Rojas-Galicia - Anal. Biochem. 152, 308 (1986) - beschrieben.

## Renaturierung der inaktiven Inhibitoren nach der Bromcyan-Spaltung

Die Renaturierung der nach Bromcyan-Spaltung erhaltenen inaktiven Inhibitoren erfolgte nach dem von Creighton erstmals für Aprotinin beschriebenen Verfahren (T.E. Creighton, in: UCLA Symposia on Molecular and Cellular Biology 39, 249-257; 1986, D.L. Oxender, Ed., A.R. Liss, Inc., New York).

## Enzymteste

## Bestimmung des Inhibitorgehaltes in Fermentationsansätzen

Eine Probe der Fermentationsbrühe wurde durch Zentrifugieren geklärt und die inhibitorische Aktivität im Überstand und in den Zellen wie folgt bestimmt:

a) Kulturfiltrat: 1 ml Kulturfiltrat wurde mit 10 $\mu$l einer 5 %igen wäßrigen Lösung von Tween 80 und 40 $\mu$l Perchlorsäure (70 %ig) unter gutem Durchmischen versetzt. Man hielt die Mischung 30 Minuten bei Raumtemperatur und trennte den gebildeten Niederschlag durch Zentrifugieren ab. Die überstehende klare Lösung wurde durch Zugabe von 180 $\mu$l gesättigter Trislösung neutralisiert.

Zu 420 $\mu$l 0,2 M Tris-Salzsäure-Puffer pH 8,0, der 0,5 % Natriumazid und 0,1 % Tween 80 enthält, fügte man 8 $\mu$l einer frisch hergestellten Lösung von humaner Granulozytenelastase - erhalten durch einhundertfache Verdünnung einer Stocklösung von 1 mg Enzym je ml 0,05 M Natriumacetatpuffer pH 5,5 (Ethylenglykol 1:1 mit Testpuffer) und in einer Konzentrationsreihe die gewünschten Volumina des neutralisierten Fällungsüberstandes. Dann wurde in jeder Probe das Volumen mit Testpuffer auf 550 $\mu$l ergänzt, wobei die 100 % - Probe nur Testpuffer enthielt. Nach 30 Minuten Vorinkubation bei Raumtemperatur wurde mit jeweils 100 $\mu$l einer Mischung von Testpuffer und 6,5 $\mu$l einer 0,1 M Lösung von MeOSuc-Ala-Ala-Pro-Val-pNA versetzt und der durch die enzymatische Freisetzung von p-Nitroanilin bedingte Extinktionsanstieg bei 405 nm bestimmt. Die prozentuale Inhibition errechnet sich nach:

$$\% \text{ Inhibition} = 100 \times \left[ 1 - \frac{\text{OD mit Inhibitorzusatz}}{\text{OD ohne Inhibitorzusatz}} \right]$$

Die quantitative Ermittlung der Inhibitorgehalte erfolgte unter Verwendung der mit rekombinanten Val-15-Aprotinin erstellten Eichkurven.

b) Zellen: Die beim Zentrifugieren der Kulturbrühen erhaltenen Zellen wurden in 1/10-Originalvolumen 50 mM Tris-HCl-Puffer pH 7,5 aufgeschlämmt, der 0,05 %ig an Tween 80 war.

Die Zellen wurden unter Verwendung eines Ultraschallgerätes (Branson Sonifier, Cell Disruptor B 15) bei 4° C aufgeschlossen (400 Watt, 1 Minute Beschallung je ml Probenvolumen). Nach der Fällung der endogenen Inhibitoren mit Perchlorsäure in der oben beschriebenen Weise wurde der Inhibitorgehalt analog bestimmt.

Ermittlung der Hemmspezifität der Inhibitoren

Die Bedingungen für die Testung der Reinsubstanzen auf ihre inhibitorische Aktivität für die Enzyme Cathepsin G, Granulozytenelastase, Organ-(Harn)kallikrein, Plasmakallikrein und der Trypsine sind in Tabelle 1 zusammengestellt. Die Bestimmung der Enzymaktivitäten sowie die Hemmung der Enzyme wurden nach an sich bekannten Verfahren durchgeführt.

Tabelle 1

| Bedingungen für die Testung diverser humaner Serinproteinasen | | | | | |
|---|---|---|---|---|---|
| Enzym | [Eo] (M) | Testpuffer* | Substrat | [So] (M) | Vorinkubation (min) |
| Cathepsin G (human) | $2 \times 10^{-7}$ | 0,2 M Tris; 50 mM $MgCl_2$; 0,05 % Tween 80, pH 7,2 | MeOSuc-Ala-Ala-Pro-Met-pNA[1] | $1 \times 10^{-3}$ | 60 |
| Granulozytenelastase (human) | $5 \times 10^{-9}$ | 0,2 M Tris; 0,5 % Tween 80, pH 8,0 | MeOSuc-Ala-Ala-Pro-Val-pNA[1] | $3 \times 10^{-4}$ | 180 |
| Organkallikrein (human) (Harn) | $2,5 \times 10^{-8}$ | 0,2 M Tris; 0,05 % Tween 80, pH 8,2 | D-Val-Leu-Arg-pNAx2HCl[2] | $1,5 \times 10^{-4}$ | 30 |
| Plasmakallikrein (human) | $3 \times 10^{-9}$ | 0,2 M Tris; 0,05 % Tween 80, pH 8,2 | D-Pro-Phe-Arg-pNAx2HCl[3] | $4 \times 10^{-4}$ | 120 |
| Plasmin (human) | $5 \times 10^{-9}$ | 0,2 MTris/0,01M $CaCl_2$: 0,05 % Tween 80, pH 8,0 | Tos-Gly-Pro-Lys-pNA-$CH_3$-COOH[4] | $1 \times 10^{-4}$ | 90 |
| Trypsin (Rind) | $1 \times 10^{-9}$ | 0,2 M Tris; 0,01 M $CaCl_2$; 0,05 % Tween 80, pH 8,0 | Pyr-Gly-Arg-pNA[4] | $2 \times 10^{-5}$ | 90 |
| Trypsin anionisch (human) | $1 \times 10^{-9}$ | 0,2 M Tris; 0,01 M $CaCl_2$; 0,05 % Tween 80, pH 8,0 | Pyr-Gly-Arg-pNA[4] | $2 \times 10^{-5}$ | 90 |
| Trypsin cationisch (human) | $1 \times 10^{-9}$ | 0,2 M Tris; 0,01 M $CaCl_2$; 0,05 % Tween 80, pH 8,0 | Pyr-Gly-Arg-pNA[4] | $2 \times 10^{-5}$ | 90 |

* alle Testpuffer enthielten 0,05 % $NaN_3$

[1] K. Nakajima, J.L. Powers, B.M. Ashe und M. Zimmermann, J. Biol. Chem. 254, 4027-4032 (1979)

[2] E. Ammundsen, J.Pütter, P.Friberger, M.Knos, M.Larsbraten und M.Claeson, Adv. Exp. Med. Biol. 120A (1979)

[3] A.M. Venneröd, K. Laake, A.K. Solberg und S. Strömland, Throm. Res. 9, 457 (1976)

[4] B. Wiman, Throm. Res. 17, 143 (1980)

[5] G. Claeson et al., Haemostasis 7, 76 (1978)

EP 0 393 431 A1

## Konstruktion von Vektoren für die Expression von API-Homologen in Hefe

Das Ausgangsplasmid für die Konstruktion von pMT15 ist pCGS65 von C.A. Kaiser, Massachusetts Institute of Technology, Cambridge, MA. Das Plasmid trägt als Selektionsmarker Amp$^R$ und URA3 sowie den Replikationsorigin von pBR322 und ein Segment von 2 μ, wodurch dieses Plasmid in S.cerevisiae und E.coli replizieren kann. pCGS65 enthält die gesamte cDNA von SUC2 (Invertase). Durch BamHI-Spaltung wird das COOH-terminale Ende der Invertase deletiert und durch ein 160bp BamHI-BglII-Fragment von URA3 ersetzt, welches als Transkriptionsterminator in Hefe wirkt (Yarger et al. Mol.Cell.Biol. 6, 1095-1101; 1986). Das so entstandene Plasmid pIT18 wurde mit EcoRI und BamHI gespalten, wodurch ein 1700bp-Fragment des SUC2-Promoters und die NH$_2$-terminale Hälfte der Invertase deletiert wurde. Dieser Teil wurde ersetzt durch ein 1200bp EcoRI-BamHI-Fragment aus pJS12 (R.C. Das et al. Mol.Gen.Genet. 1989 in press), welches den MFα 1 Promoter und die pre-pro-Leadersequenz des α-Faktors enthält zusammen mit den 34 NH$_2$-terminalen Aminosäuren der Invertase. Hieraus entsteht pMT15 als Ausgangsvektor für die Expression von API-Homologen in Hefe. Die Gene der API-Homologen wurden als 180bp HindIII-Fragment (siehe Version 1, Abb. 2) in die HindIII-Schnittstelle von pMT15 kloniert.

## Hefe-Transformation

100 ml Hefezellen von SC106 (S2207A von Yeast Genetics Stock Center, University of California, Berkeley, CA 94720) werden in SD-Medium (0,67 % yeast nitrogen base without aminoacids und 2 % Glucose) supplementiert mit jeweils 20 mg/l Threonin, Methionin und Histidin bei 30° bis 2 x 10⁷ Zellen/ml gezüchtet. Die Zellen werden durch Zentrifugation geerntet, 1 x mit 5 ml TE (10 mM Tris-HCl pH 7,5, 1mM EDTA) und 1 x mit 5 ml TE + 0,1 M Lithiumacetat gewaschen. Das Zellpellet wird in 1 ml 0,1 M Lithiumacetat suspendiert und für 1 Stunde bei 30°C inkubiert. Die kompetenten Zellen können bis zu 2 Tagen bei 4°C aufbewahrt werden. 0,1 ml Zellen werden mit 10 μl Plasmid-DNA (ca. 5 μg) und 15 μl denaturierter Heringsperm-DNA (3 mg/ml) versetzt. Nach 30 Minuten bei 30°C werden 0,7 ml TE (40 % PEG 3350, 0,1 M LiOA) hinzugegeben und 1 Stunde bei 30°C inkubiert. Die Zellen werden für 5 Minuten auf 42°C erhitzt. Anschließend wird das Zellpellet 2 x mit 0,5 ml TE gewaschen. Das Zellpellet wird in 0,1 ml TE suspendiert und auf Agarplatten mit Selektionsmedium ausgestrichen. Transformanten erscheinen nach 3 Tagen.

## Kulturbedingungen

Transformierte Hefezellen werden in SD-Medium (supplementiert mit jeweils 20 mg/l Threonin, Histidin und Methionin) bei 28°C oder 30°C über einen Zeitraum von bis zu 96 Stunden fermentiert. Die Zellen werden durch Zentrifugation geerntet und die inhibitorische Aktivität der API-Homologen wird im Medium gemessen.

## Ansprüche

1) Peptid, enthaltend im wesentlichen die Aminosäuresequenz des Alzheimer Protease Inhibitors (API), wobei mindestens eine Aminosäure der natürlichen Sequenz durch eine andere natürliche Aminosäure ersetzt worden ist.

2) Peptide gemäß Anspruch 1, mit einem Aminosäureaustausch in Position 13 und/oder 15, 37, 50.

3) Peptide gemäß den Ansprüchen 1 und 2 mit einem oder mehreren der folgenden Austausche
Position 13
Val, Ile, Leu, Ala, Met, Asn, Gln, Phe, Tyr oder Trp
Position 15:
Leu, Gly, Ala, Phe, Arg, Val, Ile, Ser, Thr, Asp, Glu, Asn, Gln oder Tyr
Position 37:
Arg, Val, Ile, Leu, Met, Asn, Gln, Asp, Glu, Phe, Ser oder Thr
Position 50:

Val, Ile, Leu, Gln, Glu, Thr, Asp, Phe, Lys oder Arg.

4) Arzneimittel, enthaltend ein oder mehrere Peptide der Ansprüche 1 bis 3.

5) Verwendung der Peptide aus den Ansprüchen 1 bis 3 als Protease Inhibitoren.

6) Desoxyribonucleinsäure codierend für eines der Peptide gemäß der Ansprüche 1 bis 3.

7) Expressionsvektor enthaltend die Desoxyribonucleinsäure gemäß Anspruch 6.

EP 0 393 431 A1

FIG.1

Hind III                 Pst I
↓                       ↓

A   GCT   GAG   GTC   TGC   AGT......
     CTC   CAG   ACG   TCA......

Version 1:

    - Ala- Glu - Val - Cys - Ser -

Nhe I                Pst I                   Apa I               Eco RV
↓                 ↓                      ↓                   ↓

Version 2: CT AGC GAG GTC TGC AGT GAA CAA GCC GAG ACG GGC CCG TGC CGA GCA ATG ATA TCC
          G CTC CAG ACG TCA CTT GTT CGG CTC TGC CCG GGC ACG GCT CGT TAC TAT AGG

     Glu-Val-Cys-Ser-Glu-Gln-Ala-Glu-Thr-Gly-Pro-Cys-Arg-Ala-Met-Ile-Ser-

            Acy I                   BssH II
            ↓                      ↓

CGC TGG TAC TTT GAC GTC ACT GAA GGG AAG TGC GCG CCA TTC TTT TAC GGC GGA TGT
GCG ACC ATG AAA CTG CAG TGA CTT CCC TTC ACG CGC GGT AAG AAA ATG CCG CCT ACA
-Arg-Trp-Tyr-Phe-Asp-Val-Thr-Glu-Gly-Lys-Cys-Ala-Pro-Phe-Phe-Tyr-Gly-Gly-Cys-

            Xmn I                   Sal I                 Bam III
            ↓                      ↓                  ↓

GGC GGC AAC CGG AAC AAC TTC GAC ACA GAA GAG TAC TGC ATG GCC GTG TGT GGA
CCG CCG TTG GCC TTG TTG AAG CTG TGT CTT CTC ATG ACG TAC CGG CAC ACA CCT

Gly Gly Asn Arg Asn Asn Phe Asp Thr Glu Glu Tyr Cys Met Ala Val Cys Gly

            Hind III
            ↓

TCC GCC ATT TGA TA
AGG CGG TAA ACT ATT CGA

Ser Ala Ile xxx xxx             FIG.2

EP 0 393 431 A1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | EP-A-0 304 013 (ASAHI KASEI KOGYO K.K.) --- | | C 07 K 7/10<br>A 61 K 37/64<br>C 12 N 15/15 |
| A | EP-A-0 297 362 (BAYER AG) ----- | | |

RECHERCHIERTE SACHGEBIETE (Int. Cl.5)

C 07 K
A 61 K
C 12 N

Der vorliegende Recherchenbericht wurde für alle Patentansprüche erstellt

| Recherchenort | Abschlußdatum der Recherche | Prüfer |
|---|---|---|
| DEN HAAG | 26-07-1990 | DEFFNER C-A.E. |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus andern Gründen angeführtes Dokument
···············································································
& : Mitglied der gleichen Patentfamilie, übereinstimmendes
    Dokument

EPO FORM 1503 03.82 (P0403)